# EUROPÄISCHE PATENTSCHRIFT

(11) **EP 1 778 363 B1**
(45) Veröffentlichungstag und Bekanntmachung des Hinweises auf die Patenterteilung: **01.08.2012**
(21) Anmeldenummer: 05762744.0
(22) Anmeldetag: 16.06.2005
(51) Int. Cl.: A61K 8/04, C11D 3/20, A61K 8/86, A61K 8/39, A61K 8/36, A61K 8/34, A61K 8/23, A61Q 5/10, A61Q 5/06

(54) **STABILE GEL-FORMULIERUNGEN MIT ERHÖHTEM ELEKTROLYTGEHALT**
STABLE GEL-FORMULATION WITH INCREASED ELECTROLYTE CONCENTRATION
FORMULATIONS DE GEL STABLES A TENEUR ACCRUE EN ELECTROLYTE

(30) Priorität: 12.08.2004 DE 102004039181
(43) Veröffentlichungstag der Anmeldung: 02.05.2007
(73) Patentinhaber: Henkel AG & Co. KGaA, 40589 Düsseldorf (DE)
(72) Erfinder: GOUTSIS, Konstantin, 41812 Erkelenz (DE); MEINIGKE, Bernd, 51381 Leverkusen (DE); HÖFFKES, Horst, 40595 Düsseldorf (DE)
(86) Internationale Anmeldenummer: PCT/EP2005/006458
(87) Internationale Veröffentlichungsnummer: WO 2006/015650

(56) Entgegenhaltungen:
- WO-A-02/24155
- WO-A-03/011236
- US-A- 4 555 246
- US-A1- 2002 194 683
- US-B1- 6 660 045

## Beschreibung

Gegenstand der Erfindung sind Zusammensetzungen in Gel-Form, die einen erhöhten Gehalt an Elektrolyt enthalten und deren Verwendung als Trägermedien für oxidative Färbemittel.

Für das Färben von Keratinfasern, insbesondere von Haaren, Pelzen und Fellen spielen die sogenannten Oxidationsfärbemittel wegen ihrer intensiven Farben und guten Echtheitseigenschaften eine bevorzugte Rolle. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Eine bestimmte Entwicklersubstanz kann durch Kombination mit unterschiedlichen Kupplern auch sehr unterschiedliche Farbnuancen bilden. Trotzdem gelingt es oft nicht, mit Hilfe einer einzigen Entwicklersubstanz zu einer Vielzahl natürlicher Farbnuancen zu kommen. In der Praxis ist daher meist eine Kombination verschiedener Entwicklerkomponenten und Kupplerkomponenten erforderlich, um eine einzige, natürlich wirkende Färbung zu erhalten.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Bei ihrer Anwendung in Haarfärbemitteln sollen sie bereits bei Temperaturen unterhalb 40°C ein gutes Aufziehvermögen auf menschlichem Haar besitzen, wobei keine merklichen Unterschiede zwischen strapaziertem und frisch nachgewachsenem Haar bestehen dürfen (Egalisiervermögen). Sie sollen beständig sein gegen Licht, Wärme und den Einfluß von Haarshampoos und chemischen Reduktionsmitteln, z. B. gegen Dauerwellflüssigkeiten. Schließlich sollen sie die Kopfhaut nicht zu sehr anfärben und vor allem sollen sie in toxikologischer und dermatologischer Hinsicht unbedenklich sein.

Ein vielseits bewährtes Oxidationsfarbstoffvorprodukt ist das als Entwicklerkomponente verwendete p-Toluylendiamin, das in toxikoligischer Hinsicht deutlich günstiger zu bewerten ist als p-Phenylendiamin. p-Toluylendiamin ist in Oxidationshaarfärbemittel-Zubereitungen bislang nur in elektrolytischer Form seiner Salze, z.B. der Hydrochloride, Hydrobromide oder Sulfate stabil einarbeitbar. In Form der freien Base ist p-Toluylendiamin (bei 25°C) eine oxidationsempfindliche schwer handhabbare Flüssigkeit, deren Einarbeitung in Färbezubereitungen auch in Gegenwart von Antioxidationsmitteln kaum zu bewerkstelligen ist. ("Freie Base" im Sinne der vorliegenden Erfindung bedeutet, daß weniger als 20 % der Aminofunktionen von Mono- oder Diaminen in Salzform vorliegen.) p-Phenylendiamin hingegen ist in Form der freien Base bei 25°C ein kristalliner, verhältnismäßig oxidationsstabiler Feststoff.

Oxidationshaarfärbemittel in Gel-Form werden vom Verbraucher als besonders ästhetische Anbietungsform empfunden. Der Begriff "Gel" wird in "Faraday discussions of the chemical society, No. 57, 1974 (Gels and Gelling Processes), P.J. Flory, Seite 7-18", näher charakterisiert. Demnach werden Gele in 4 Untergruppen eingeteilt:
1. Gele mit lamellaren Ordnungsstrukturen einschließlich Gel-Mesophasen, z.B. Tensidgele, insbesondere Seifengele.
2. Gele, die aus polymeren, dreidimensionalen, kovalent gebundenen Netzwerken, die ohne Zerstörung der Kovalenzbindungen in Lösungsmitteln unlöslich sind, aufgebaut sind.
3. Gele aus polymeren Netzwerken mit lokalen Ordnungsstrukturen, die durch physikalische Kräfte zusammengehalten werden, z.B. Gelatine, Homo- und Copolymere von Acryl- und Methacrylsäure, Alginate, Carrageenan.
4. Gele gebildet aus feinverteilten Bestandteilen, z.B. Niederschlägen, die i.a. eine große geometrische Anisotropie aufweisen, z.B. V₂O₅-Gele oder Gele, die sich durch Aggregation von Proteinen bilden.

Gele im Sinne der nachfolgend beschriebenen Erfindung sind solche, die unter Punkt 1. und Punkt 3. der obigen Auflistung fallen. Als "Gel" im Sinne der vorliegenden Erfindung sind auch Transparentgele wie sie z.B. in den US-Patentschriften US 3 101 300 und US 3 101 301 beschrieben werden, zu verstehen. Diese Transparentgele werden als Mikroemulsionsgele bezeichnet. Eine allgemeingültige Definition für Mikroemulsionsgele ist in "Happi: Household Pers. Prod. Ind. 30 (1993), Nr. 2, p. 58-64" angegeben.

Das Gel sollte nicht notwendigerweise, aber vorzugsweise fließfähig sein, und durch eine hohe Strukturviskosität gekennzeichnet sein, d.h., diese Zubereitungen erscheinen unter dem Einfluß geringer Scherkräfte, etwa des Eigengewichts, relativ hochviskos, lassen sich aber bei Anwendung höherer Scherkräfte, z.B. beim Verrühren sehr leicht bewegen. Besonders wichtig ist, daß nach dem Vermischen mit einer die oxidative Haarfärbung bewirkenden wäßrigen Oxidationsmittel-Zubereitung die Strukturviskosität des Färbeansatzes erhalten bleibt und der gebrauchsfertige Färbeansatz sich zwar leicht auf dem Haar verteilen läßt, nicht aber vom Haar abläuft und die Kopfhaut oder das Gesicht anfärbt.

Im Vergleich zu Oxidationshaarfärbemitteln in Creme-Form bieten Färbemittel in Gel-Form einige anwendungstechnische Vorteile. So ist die Herstellung von Cremes (i.a. O/W-Emulsionen) generell problematischer und aufwendiger; Cremes dicken oft nach, was für 2-Komponenten-Verpackungen besonders nachteilig ist. Die in den Rahmen der Erfindung fallenden Gele hingegen behalten ihre einmal eingestellte Viskosität über einen längeren Zeitraum bei und lassen sich leichter auf dem Kopfhaar verteilen.

Es hat sich nun gezeigt, daß Oxidationshaarfärbemittel in Gel-Form bei Anwesenheit von Elektrolyten, insbesondere von Hydrochloriden, Hydrobromiden oder Sulfaten des p-Toluylendiamins, unbeständig sind. Es tritt meist eine Phasentrennung auf, die nicht reversibel ist. So bilden sich z.B. entweder eine trübe und eine klare Phase oder zwei klare Phasen unterschiedlicher Viskosität, die durch eine Phasengrenzfläche voneinander getrennt sind.

Im Stand der Technik finden sich zwar Beispiele für Oxidationshaarfärbemittel in Gel-Form, die Elektrolyte, insbesondere p-Toluylendiamin als Salz, enthalten; sämtliche Zubereitungen sind jedoch nicht zufriedenstellend aufgrund von Phasentrennung oder wegen der schwierigen Einarbeitbarkeit des p-Toluylendiamins in Form der freien Base.

So werden in den deutschen Offenlegungsschriften DE 33 02 534 (Beispiel 41) und DE 34 21 694 (Beispiele 27, 41, 42) Oxidationshaarfärbemittel in Gel-Form offenbart, die p-Toluylendiamin-dihydrochlorid enthalten.

Die deutsche Offenlegungsschrift DE 40 22 847 beschreibt ein gelförmiges Oxidationshaarfärbemittel, in dem (als freie Base vorliegendes) p-Toluylendiamin und als Antioxidationsmittel Ascorbinsäure und Natriumsulfit enthalten sind.

In "Hair dyes, J.C. Johnson, Noyes Data Corporation 1973, Seiten 313 - 330" werden Gel-Oxidationshaarfärbemittel mit p-Toluylendiamin offenbart, die entweder das Antioxidationsmittel Natriumbisulfit oder kein Antioxidationsmittel enthalten. All diese im Stand der Technik offenbarten Rezepturen verfügen jedoch nicht über die gewünschte Stabilität.

In der europäischen Patentschrift EP-B1-1 035 825 werden fließfähige gelförmige Träger für oxidative Färbemittel offenbart. Diese Träger sind jedoch bei erhöhtem Elektrolytgehalt nicht lagerstabil.

Aufgabe der vorliegenden Erfindung ist es daher, eine leicht handhabbare gelförmige Zusammensetzung in lagerstabiler Form bereitzustellen, die eine erhöhte Menge an Elektrolyt enthalten kann, so dass Wirkstoffe, wie zum Beispiel die Oxidationsfarbstoffvorprodukte (insbesondere p-Toluylendiamin), in der Salzform stabil eingearbeitet werden können.

Es wurde nun überraschenderweise gefunden, dass die Gegenwart von ethoxylierten Fettalkoholen mit einem Ethoxylierungsgrad von 15 bis 100 Einheiten Ethylenoxid pro Molekül Fettalkohol ein gelförmige elektrolythaltige Zusammensetzung in ihrer Gelform stabilisiert. Die Zusammensetzungen besitzen hervorragende Eigenschaften in der Anwendung, insbesondere am Haar. Wenn die Zusammensetzungen als Trägermedium für oxidative Haarfärbemittel Anwendung finden, erlauben sie haltbare und intensive oxidative Färbungen.

Gegenstand der Erfindung ist eine gelförmige Zusammensetzung, insbesondere als Trägermedium für Oxidationsfarbstoffvorprodukte, enthaltend
- 0,5 bis 10 Gew.-% eines Anlagerungsproduktes von 15 bis 100 Einheiten Ethylenoxid pro Molekül eines linearen Fettalkohols mit 8 bis 22 C-Atomen,
- 0,5 bis 10 Gew.-% eines Anlagerungsproduktes von 1 bis 5 Mol Ethylenoxid an einen Ilnearen Fettalkohol mit 8 bis 22 C-Atomen,
- 6,0 bis 15 Gew.-% eines gesättigten oder ungesättigten, linearen oder verzweigten Alkohols mit 8 bis 36 C-Atomen,
- 0,1 bis 15 Gew.-% einer flüssigen Fettsäuren mit 16 bis 22 C-Atomen in Form einer wasserlöslichen Seife,
- 0 bis 20 Gew.-% eines niedermolekularen wasserlöslichen Alkohols und
- nicht weniger als 12 mmol eines Elektrolyts bezogen auf 100g der gelförmigen Zusammensetzung, dadurch gekennzeichnet, dass sie eine Viskosität von höchstens 100 mPa.s, gemessen bei 20°C mit einem Rotationsviskosimeter Brookfield, Typ RTV, Spindel#4, bei 4 rom. besitzt:

Bei den erfindungsgemäßen Elektrolyten handelt es sich definitionsgemäß um Salze anorganischer oder organischer Verbindungen, welche bevorzugt durch Reaktion einer anorganischen oder organischen Säure mit einer anorganischen oder organischen Base gebildet werden Unter einem Salz wird erfindungsgemäß eine heteropolare Verbindung verstanden, an deren Kristallgitter mindestens eine von Wasserstoffionen (Protonen) verschiedene Kationenart und mindestens eine von Hydroxidionen verschiedene Anionenart beteiligt sind. Dabei werden starke Elektrolyte erfindungsgemäß bevorzugt.

Diese werden aus schwachen Basen mit starken Säuren, bzw. starken Basen mit schwachen Säuren gebildet.

Es ist besonders bevorzugt, wenn die Elektrolyten ausgewählt werden aus der Gruppe, die gebildet wird aus,
- anorganischen Salzen, wie beispielsweise Natriumchlorid, Natriumsulfat, Ammoniumsulfat, Natriumsulfit, und Calciumchlorid,
- Salzen, die durch Umsetzung organischer Verbindungen mit organischen oder anorganischen Säuren, insbesondere anorganischen Säuren, gebildet werden, wie z.B. p-Toluylendiamin H₂SO₄, 2,4,5,6-Tetraaminopyrimidin 2 H₂SO₄ Diese Salze haben erfindungsgemäß keinen tensidischen Charakter und keine Emulgatoreigenschaften.

Elektrolyt sind in einer Menge von nicht weniger als 12 mmol, insbesondere nicht weniger als 15 mmol jewells bezogen auf 100 g der erfindungsgemäßen gelförmigen Zusammensetzung, enthalten. Bevorzugt wird die Menge eines Elektrolyts als Produkt von der Wertigkeit des Elektrolyts (Ladungszahl) multipliziert mit der Stoffmenge des Elektrolyts in Mol angegeben. Dieses Produkt trägt die Einheit Val. Es ist bevorzugt, dass Elektrolyte in einer Menge von mehr als 25 Millival (mVal), insbesondere mehr als 30 mVal, jeweils bezogen auf 100 g der erfindungsgemäßen, gelförmigen Zusammensetzung, enthalten ist.

Es wird angestrebt, daß die gelförmige Zusammensetzung fließfähig ist sie besitzt eine Viskosität von höchstens 100 mPa·s (20°C), bevorzugt von höchstens 20 mPa·s (20°C). Wird die erfindungsgemäße gelförmige Zusammensetzung als Trägermedium für Oxidationshaarfärbemittel verwendet, welst der anwendungsfertige Färbeansatz, der durch Vermischen der erfindungsgemäßen Zusammensetzung mit einer Oxidationsmittelzubereitung entsteht, bevorzugt eine Viskosität von wenigstens 3 Pa·s (20°C), bevorzugt von 8 bis 20 Pa·s (20°C), auf (Viskositätsmessungen jeweils mit dem Rotationsviskosimeter Brookfield, Typ RTV, Spindel 4, 4 rpm).

In einer bevorzugten Ausführungsform ist die erfindungsgemäße Zusammensetzung ein klares Gel. Ein Gel ist als nicht klar zu bewerten, wenn bei einer Schichtdicke von 1 cm die Extinktion bei einer Wellenlänge, in dem die gelförmige Zusammensetzung keine Absorptionsbanden aufweist (typischerweise bei einer Wellenlänge von 800 nm) >0.05 Extinktionseinhelten beträgt.

Es ist erfindungsgemäß, Anlagerungsprodukten von 15 bis 100 Mol Ethylenoxid, insbesondere von 15 bis 50 Mol Ethylenoxid, an einen linearen Fettalkohol mit 8 bis 22 Kohlenstoffatomen in den gelförmigen Zusammensetzungen zu verwenden. Es handelt sich dabei ganz besonders bevorzugt um Ceteareth-15 oder Ceteareth-50, welche als Eumulgin^{®} CS 15 bzw. Eumulgin^{®} CS 50 von der Firma Cognis Deutschland GmbH vermarktet werden.

Bei den erfindungsgemäß einzusetzenden gesättigten oder ungesättigten, linearen oder verzweigten Alkoholen mit 8 bis 36 C-Atomen handelt es sich vorzugsweise um Fettalkohole und/ oder Guerbetalkohole.

Unter Fettalkoholen sind primäre aliphatische Alkohole der Formel (I) zu verstehen,

R⁷OH (I)

in der R⁷ für einen aliphatischen, linearen oder verzweigten Kohlenwasserstoffrest mit 8 bis 22 Kohlenstoffatomen, der gesättigt ist oder bis zu 3 Doppelbindungen enthalten kann, steht.

Typische Beispiele sind 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Bevorzugt sind technische Fettalkoholmischungen mit 12 bis 18 Kohlenstoffatomen wie beispielsweise Kokos-, Palm-, Palmkern- oder Talgfettalkohol, insbesondere Kokos- und/oder Talgfettalkohol.

Unter Guerbetalkoholen sind Alkohole zu verstehen, die durch alkalische Kondensation von Alkoholen zu höhermolekularen, verzweigten Iso-Alkoholen hergestellt werden. Diese Umsetzung wurde erstmals von Guerbet 1899 veröffentlicht. Machemer stellte 1952 wesentliche Schritte der Reaktion dar (Angewandte Chemie 64 (1952) 213 - 20): Neben der Dehydrierung zum Keton, bei der Wasserstoff abgespalten wird, und der Aldolkondensation ist die Crotonisierung, bei der Wasser abgespalten wird, ein wichtiger Schritt im Reaktionsablauf. Stand der Technik ist eine Reaktionsführung bei Normaldruck und einer Reaktionstemperatur von 240 bis 260 °C. Die so erhaltenen verzweigten Alkohole werden als Guerbetalkohole bezeichnet. Aus dem Stand der Technik sind inzwischen eine Vielzahl weiterer Verfahren bekannt, gemäß derer man Guerbetalkohole erhalten kann.

Unter niedermolekularen Alkoholen sind im Sinne der vorliegenden Erfindung mit Wasser mischbare Alkohole mit 1 bis 5 C-Atomen zu verstehen. Vorzugsweise handelt es sich um Ethanol, Propanol und/ oder Isopropanol.

Die zur Seifenbildung geeigneten Fettsäuren sind bevorzugt flüssige oder niedrigschmelzende ungesättigte lineare C₁₆-C₂₂-Fettsäuren wie Palmitoleinsäure, Ölsäure, Elaidinsäure, Myristinsäure, Petroselinsäure, Petroselaidinsäure, Gadoleinsäure, Erucasäure, Brassidinsäure sowie Gemische dieser Fettsäuren untereinander und gegebenenfalls mit geringeren Anteilen gesättigter linearer Fettsäuren mit 12 bis 22 C-Atomen. Andere ebenfalls geeignete Fettsäuren sind verzweigte Fettsäuren mit 16 bis 22 C-Atomen, z.B. die 2-Hexyldecansäure, Isostearinsäure und die 2-Octyldodecansäure.

Zur Überführung der Fettsäuren in wasserlösliche Seifen eignen sich Alkalihydroxide und Alkalicarbonate, Ammoniak, Mono-, Di- und Trialkanolamine mit 2 bis 4 C-Atomen in der Alkanolgruppe.sowie alkalische Aminosäuren wie beispielsweise Arginin, Ornithin, Lysin und/ oder Histidin.

Als Polyole sind insbesondere solche mit 2 bis 6 C-Atomen bevorzugt. Es eignen sich z.B. Ethylenglycol, 1,2-Propylenglycol, 1,3-Propylenglycol, Glycerin, Erythrit, Trimethylolpropan, Diethylenglycol und Dipropylenglycol. 1,2-Propylenglycol ist besonders bevorzugt.

Als wasserlösliche synthetische Tenside eignen sich bevorzugt anionische, amphotere, zwitterionische und nichtionische Tenside mit guter Wasserlöslichkeit und mit gutem Kalkseifendispergiervermögen. Solche Tenside weisen in der Regel eine lipophile lineare Alkyl- oder Acylgruppe mit 12 bis 18 C-Atomen und eine stark dissoziierte ionische Gruppe oder eine nichtionische wasserlöslichmachende Polyethergruppe auf. Geeignet sind z.B. Schwefelsäurehalbestersalze von linearen Fettalkoholen mit 12 bis 18 C-Atomen oder von Fettalkoholpolyglycolethern mit 12 bis 16 C-Atomen in der Alkylgruppe und 1 bis 10 Glycolethergruppen. Weitere geeignete Aniontenside sind z.B. lineare Alkansulfonate und α-Olefinsulfonate mit 12 bis 18 C-Atomen. Geeignete nichtionogene Tenside sind z.B. die Anlagerungsprodukte von 7 bis 30 Mol Ethylenoxid an lineare Fettalkohole mit 12 bis 18 C-Atomen, an Fettsäuren mit 12 bis 18 C-Atomen sowie an Fettsäuremonoglyceride und an Fettsäure-sorbitanmonoester. Bevorzugt geeignete nichtionogene Tenside sind die Fettalkylaminoxide und insbesondere die Fettalkylglykoside, vorzugsweise Fettalkylglucoside. Die Fettalkylgruppe kann in den genannten Produkten 12 bis 18 C-Atome aufweisen. Besonders bevorzugt im Sinne der vorliegenden Erfindung sind auch amphotere Tenside wie beispielsweise N-Fettalkyldimethyl-glycin oder N-Fettalkylaminopropionsäure und/ oder zwitterionische Tenside, z.B. N-Fettalkyl-dimethylammoniumglycinat oder N-Fettacylaminopropyldimethylglycinat. Weiterhin bevorzugt werden Kationtenside wie wie quartäre Ammoniumverbindungen (QAV) insbesondere quaternierte Trialkylammoniumverbindungen mit Alkylresten einer Kettenlänge von C8 bis C22.

Es hat sich gezeigt, daß durch den Zusatz weiterer, begrenzt wasserlöslicher, nichtionogener Tenside eine Verdickung der Oxidationsfärbemittel-Zubereitung und insbesondere des unmittelbar vor der Anwendung hergestellten Färbeansatzes erreicht wird.

Als Anlagerungsprodukte von 1 bis 4 bzw. 15 bis 100 Mol Ethylenoxid an einen linearen Fettalkohol mit 12 bis 22 C-Atomen eignen sich alle nach den bekannten technischen Oxethylierungsverfahren erhältlichen Addukte. Bevorzugt sind die Anlagerungsprodukte, die nur wenig freien Fettalkohol enthalten und eine eingeengte Homologenverteilung aufweisen (sogenannte "narrow range ethoxylates"), wie sie z.B. nach dem in DE 38 43 713 A1 beschriebenen Verfahren zugänglich sind.

Zusätzlich können die erfindungsgemäßen Mittel Anlagerungsprodukte von 1 bis 4 Mol Ethylenoxid an ein Fettalkylamin enthalten. Als Anlagerungsprodukte von 1 bis 4 Mol Ethylenoxid an ein lineares Fettalkylamin mit 12 bis 22 C-Atomen eignen sich alle nach bekannten technischen Verfahren zugänglichen Addukte, die auch im Handel erhältlich sind. Besonders geeignet ist das Anlagerungsprodukt von 2 Mol Ethylenoxid an ein C₁₂-C₁₈-Kokosalkylamin.

Geeignete Verbindungen der Formel II

R⁵-(OC₂H₄)ₓ-A-(C₂H₄O)y-R⁶ (II)

in welcher A ein Sauerstoffatom ist, sind Dialkylether mit 12 bis 18 C-Atomen in den Alkylgruppen. Solche Produkte sind literaturbekannt. Noch besser geeignet sind die Produkte der Formel II, in welchen x oder y oder beide = 1 sind. Solche Dialkyloxethylether lassen sich durch literaturbekannte Veretherungsverfahren aus Fettalkoholen und Fettalkyloxyethanolen herstellen. Die Produkte der Formel II, in welchen A eine Gruppe 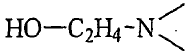 ist, lassen sich z.B. aus Triethanolamin durch O-Alkylierung mit 2 Mol eines Schwefelsäurehalbestersalzes eines C₁₂-C₂₂-Fettalkohols nach dem in DE 35 04 242 beschriebenen Verfahren zur Herstellung von Etheraminen gewinnen.

Besonders bevorzugte Verbindungen der Formel II sind z.B. Dicetylstearylether, Dicetylstearyldioxyethylether und N,N-Bis-(2-cetyl-/stearyl-oxyethyl)-aminoethanol.

Während mit Anlagerungsprodukten von 1 bis 5 Mol Ethylenoxid an einen linearen Fettalkohol die erforderliche Verdickung meist allein durch diese Komponente erreicht wird, kann es bei Einsatz der Anlagerungsprodukte von 1 bis 4 Mol Ethylenoxid an ein lineares Fettalkylamin vorteilhaft sein, diese in Kombination mit 1 bis 10 Gew.-% einer Verbindung der Formel II zu verwenden.

Insbesondere bevorzugt sind in den erfindungsgemäßen Zusammensetzungen die Proteine und/ oder -derivate pflanzlicher oder tierischer Herkunft wie beispielsweise Erbsen-, Soja-, Weizen- und Mandelproteinhydrolysat oder Akazienprotein sowie Collagen- oder Keratinhydrolysat enthalten.

In einer ersten bevorzugten Ausführungsform enthält die erfindungsgemäße Zusammensetzung mindestens eine Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (Ent1) 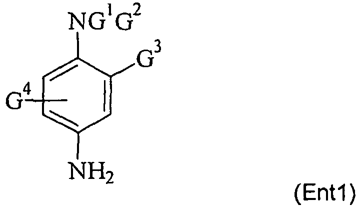 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁-bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (Ent1) sind insbesondere die Aminogruppen, C₁-bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (Ent1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen. Erfindungsgemäß ganz besonders bevorzugte p-Phenylendiaminderivate der Formel (Ent1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbemitteln gemäß der Erfindung verwendet werden können, kann man insbesondere die Verbindungen nennen, die der folgenden Formel (Ent2) entsprechen, sowie ihre physiologisch verträglichen Salze: 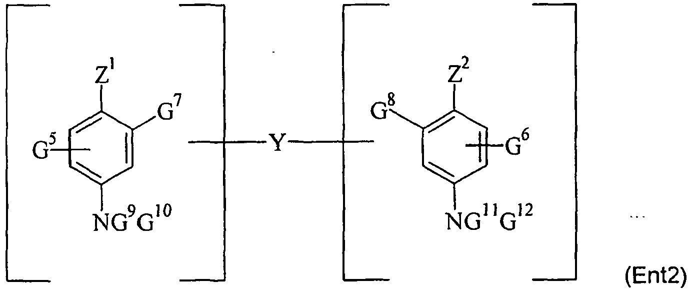 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹, und G¹² stehen unabhängig voneinander für ein Wasserstoffatom, eine direkte Bindung zur Verbrückung Y oder einen C₁- bis C₄-Alkylrest,
mit den Maßgaben, dass
- die Verbindungen der Formel (Ent2) nur eine Verbrückung Y pro Molekül enthalten und
- die Verbindungen der Formel (Ent2) mindestens eine Aminogruppe enthalten, die mindestens ein Wasserstoffatom trägt.

Die in Formel (Ent2) verwendeten Substituenten sind erfindungsgemäß analog zu den obigen Ausführungen definiert.

Bevorzugte zweikernige Entwicklerkomponenten der Formel (Ent2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (Ent2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (Ent3) 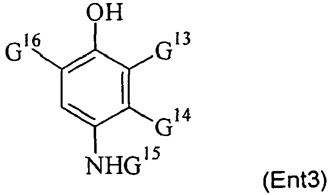 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁-bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁-bis C₄)-Alkoxy-(C₁- bis C₄)-alkylrest, einen C₁- bis C₄-Aminoalkylrest oder einen C₁-bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (Ent3) verwendeten Substituenten sind erfindungsgemäß analog zu den obigen Ausführungen definiert.

Bevorzugte p-Aminophenole der Formel (Ent3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(ß-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (Ent3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)amino-3-amino-pyridin, 2,3-Diamino-6-methoxy-pyridin, 2-(ß-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(ß-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(ß-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(ß-hydroxyethyl)amino-1-methylpyrazol.

Bevorzugte Pyrazolopyrimidin-Derivate sind insbesondere die Derivate des Pyrazolo[1,5-a]opyrimidin der folgenden Formel (Ent4) und dessen tautomeren Formen, sofern ein tautomeres Gleichgewicht besteht: 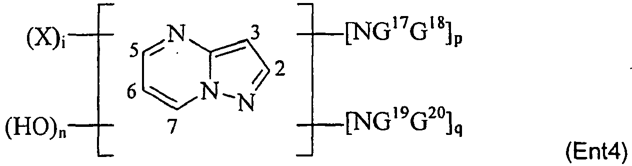 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁-bis C₄-hydroxyalkyl)aminoalkylrest, einen Aminorest, einen C₁- bis C₄-Alkyl- oder Di-(C₁- bis C₄-hydroxyalkyl)aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (Ent4) verwendeten Substituenten sind erfindungsgemäß analog zu den obigen Ausführungen definiert.

Wenn das Pyrazolo[1,5-a]pyrimidin der obenstehenden Formel (Ent4) eine Hydroxygruppe an einer der Positionen 2, 5 oder 7 des Ringsystems enthält, besteht ein tautomeres Gleichgewicht, das zum Beispiel im folgenden Schema dargestellt wird:

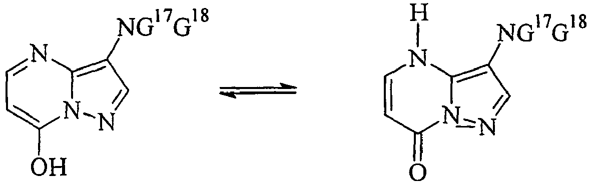

Unter den Pyrazolo[1,5-a]pyrimidinen der obenstehenden Formel (Ent4) kann man insbesondere nennen:
- Pyrazolo[1,5-a]pyrimidin-3,7-diamin;
- 2,5-Dimethyl-pyrazolo[1,5-a]pyrimidin-3,7-diamin;
- Pyrazolo[1,5-a]opyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1-,5-a]pyrimidin-3-ylamino)-ethanol;
- 2-[(3-Aminopyrazolo[1,5-a]pyrimidin-7-yl)-(2-hydroxy-ethyl)amino]-ethanol;
- 2-[(7-Aminopyrazolo[1,5-a]pyrimidin-3-yl)-(2-hydroxy-ethyl)amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;
sowie ihre physiologisch verträglichen Salze und ihre tautomeren Formen, wenn ein tautomers Gleichgewicht vorhanden ist.

Die Pyrazolo[1,5-a]pyrimidine der obenstehenden Formel (Ent4) können wie in der Literatur beschrieben durch Zyklisierung ausgehend von einem Aminopyrazol oder von Hydrazin hergestellt werden.

Als Vorstufen naturanaloger Farbstoffe werden bevorzugt als Oxidationsfarbstoffvorprodukt vom Entwicklertyp solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (IIIa), 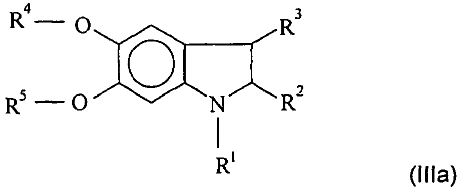 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIIb), 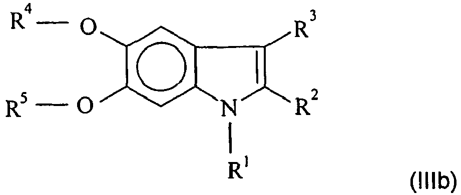 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

In einer weiteren bevorzugten Ausführungsform enthalten die erfindungsgemäßen Färbemittel mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Wenn das erfindungsgemäße Mittel als farbgebende Komponente eine Kupplerkomponente enthält, werden als erfindungsgemäß bevorzugte Kupplerkomponenten verwendet:
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)aminobenzot,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Bevorzugte direktziehende Farbstoffe, die in den erfindungsgemäßen Mitteln Verwendung finden, sind Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

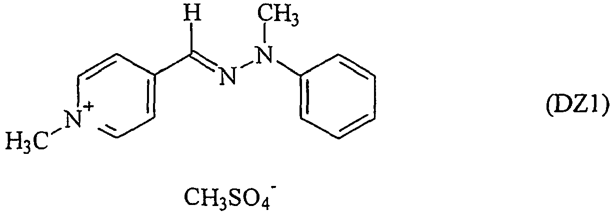

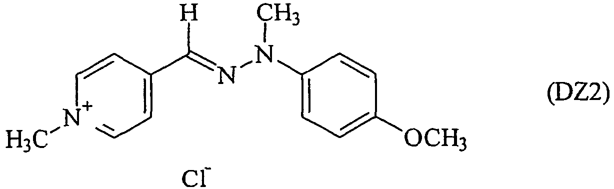

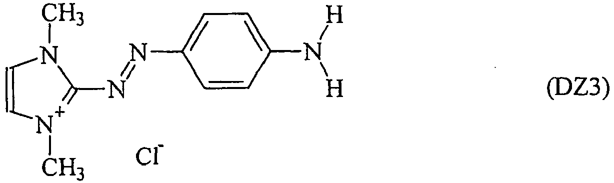

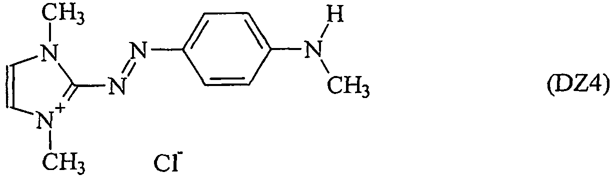

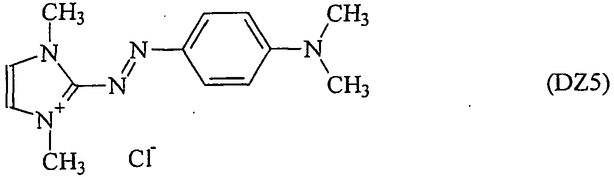

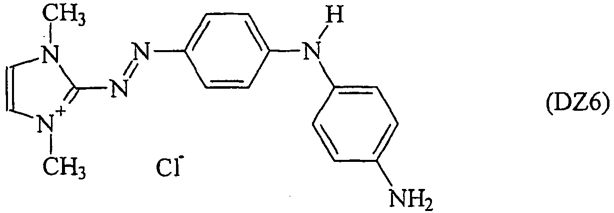

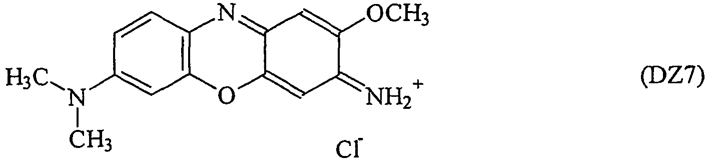

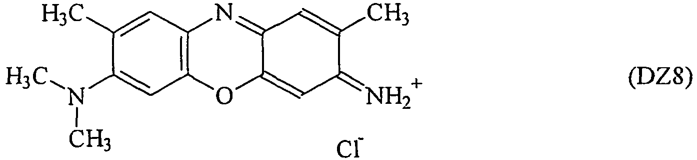

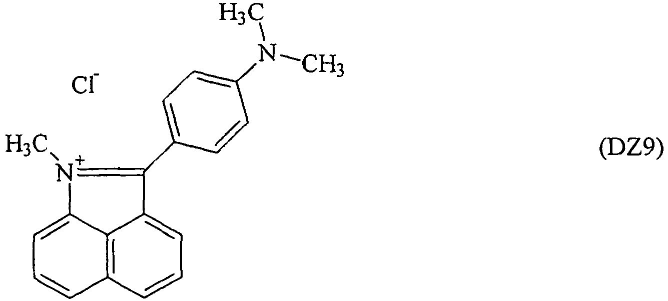

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Zusammensetzungen gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Zusammensetzungen, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Der erfindungsgemäßen Zusammensetzung können Oxidationsfarbstoffvorprodukte bzw. Direktfarbstoffe unmittelbar vor der Anwendung zugemischt werden. Es ist bevorzugt, wenn die erfindungsgemäße Zusammensetzung derart konfektioniert ist, dass zumindest Oxidationsfarbstoffvorprodukte im Gemisch mit der erfindungsgemäßen Zusammensetzung in einem Container aufbewahrt werden.

Die Oxidationsfarbstoffvorprodukte, insbesondere p-Toluylendiamin, werden in den erfindungsgemäßen Zusammensetzungen bevorzugt in Form ihrer physiologisch verträglichen Salze eingesetzt. Diese werden durch Umsetzung des Oxidationsfarbstoffvorprodukts mit einer anorganischen oder organischen Säure gebildet.

Bezüglich der in den erfindungsgemäßen Zusammensetzungen einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Als zusätzlicher Verdicker können erfindungsgemäß Xanthan-Gum, Agar-Agar, lineare und vernetzte Polyacrylate, nichtionogene und anionische Cellulosederivate in den erfindungsgemäßen Zusammensetzungen enthalten sein.

Weiterhin können haarkosmetische Hilfsstoffe enthalten sein, insbesondere Bisabolol, Pflanzenextrakte, Vitamine wie vorzugsweise Niacinamid, Tocopherol, Vitamin A, Biotin und Vitamin D.

Wenn die erfindungsgemäße Zusammensetzung Oxidationsfarbstoffvorprodukte enthält, kann die eigentliche oxidative Färbung der Fasern grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, lodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Ein zweiter Gegenstand der Erfindung ist ein oxidatives Haarfärbemittel. Dieses wird unmittelbar vor der Anwendung durch Mischung der Zubereitung des Oxidationsmittels mit der erfindungsgemäßen Zusammensetzung, enthaltend mindestens ein Oxidationsfarbstoffvorprodukt in Form von Entwickler- und/oder Kupplerkomponenten, im Gewichtsverhältnis von 1:2 bis 2:1 hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von in der Regel 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die Oxidationsmittelzubereitung enthält besonders bevorzugt

| | |
|---|---|
| 3 bis 12 Gew.-% | Wasserstoffperoxid |
| 0,1 bis 5 Gew.-% | eines wasserlöslichen, synthetischen Tensids und |
| 1 bis 5 Gew.-% | eines dispergierten Acrylsäure- und/oder Methacrylsäure-Polymerisats oder -Copolymerisats |

im wäßrigen Träger neben den in solchen Zubereitungen üblichen Stabilisierungshilfsmitteln. Sie kann aber auch im einfachsten Fall allein aus Wasser bestehen, so daß die Oxidation durch den Luftsauerstoff herbeigeführt wird.

Als wasserlösliche synthetische Tenside können in der Oxidationsmittelzubereitung die bereits für die als Trägermedium fungierende erfindungsgemäße Zusammensetzung genannten anionischen, amphoteren, zwitterionischen und nichtionischen Tenside oder Gemische davon verwendet werden. Bevorzugt werden anionische Tenside, z.B. Schwefelsäurehalbestersalze von linearen Fettalkoholen mit 12 bis 18 C-Atomen oder von Fettalkoholpolyglycolethern mit 12 bis 16 C-Atomen in der Alkylgruppe und 1 bis 10 Glycolethergruppen in Form ihrer Alkali-, Magnesium-, Ammonium- oder Alkanolammoniumsalze eingesetzt.

Die Oxidationsmittelzubereitung enthält außerdem bevorzugt Komplexbildner und Puffersalze zur Einstellung eines pH-Wertes von 2 bis 5. In diesem schwach sauren Medium bleiben die Acrylsäure- und/oder Methacrylsäure-Polymerisat-Dispersionen dünnflüssig und stabil. Beim Vermischen mit des alkalischen erfindungsgemäßen Trägermediums, das Ammoniak und Puffersalze zur Einstellung eines pH-Wertes von 8 bis 10 enthält, steigt der pH-Wert der Mischung an und die Carboxylgruppen des Polymerisats oder Copolymerisats werden in die Salzform überführt. Dabei beginnen die Polymerisate sich im wäßrigen Medium zu lösen und die Viskosität der Lösung anzuheben.

Besonders günstig für den Viskositätsaufbau nach dem Vermischen der erfindungsgemäßen Zusammensetzung und der Oxidationsmittelzubereitung ist der Gehalt an dispergierten Acrylsäure- und/oder Methacrylsäure-Polymerisat oder - Copolymerisat in der Oxidationsmittelzubereitung. Solche Dispersionen von Copolymerisaten, z.B. aus wenigstens 10 Gew.-% Acrylsäure-Niedrigalkylester, 25 bis 70 Gew.-% Methacrylsäure und ggf. bis zu 40 Gew.-% eines weiteren Comonomeren, sind z.B. in GB 870 994 beschrieben. Aus DE 11 64 095 sind Mischpolymerisate aus 50 bis 75 Gew.-% Ethylacrylat, 25 bis 35 Gew.-% Acrylsäure und 0 bis 25 Gew.-% anderer Comonomerer bekannt. Geeignete Dispersionen dieser Art sind im Handel erhältlich, z.B. unter der Handelsbezeichnung Latekoll^{®} D (BASF). Besonders gut eignen sich die in DE 34 45 549 beschriebenen Copolymerisate aus 50 bis 60 Gew.-% Ethylacrylat, 30 bis 40 Gew.-% Methacrylsäure und 5 bis 15 Gew.-% Acrylsäure, vernetzt mit Ethylenglycoldimethacrylat.

Die erfindungsgemäßen Haarfärbemittel können zusätzlich alle auf diesen Gebieten bekannten und üblicherweise verwendeten Wirk- und Hilfsstoffe enthalten.

Die erfindungsgemäßen Haarfärbemittel weisen bevorzugt einen pH-Wert von 5 bis 11, insbesondere von 8 bis 10, auf.

Zusätzlich können Polymere, beispielsweise zwitterionische und/ oder nichtionische Polymere wie Silikonöle, bevorzugt kationische Polymere wie Polymer JR 400, Merquat 100, Gafquat 734, Gafquat 755, Mirapol A15, Hexadimethrinchlorid (Kondensation von N,N,N',N'-Tetramethylhexamethylendiamin und Trimethylenchlorid) und Polyquaternium-34, enthalten sein.

Die folgenden Beispiele sollen den Gegenstand der Erfindung näher erläutern:

### Beispiele

Alle Mengenangaben entsprechen, wenn nicht anders gekennzeichnet, Gewichtsprozent der einzelnen Komponenten bezogen auf das Gewicht der entsprechenden Zusammensetzung.

Es wurden die Trägermedien gemäß Tabelle 1 hergestellt. Anschließend wurden diese bei einer Temperatur von 45°C über einen Zeitraum von 24 Stunden gelagert (Lagerung).

**Tabelle 1:**

| | E1 | E2 | E3 | E4 | V1 | V2 |
|---|---|---|---|---|---|---|
| Isopropanol | 14,5 | 14,5 | 14,5 | 14,5 | 14,5 | 14,5 |
| Ölsäure | 6,75 | 6,75 | 6,75 | 6,75 | 6,75 | 6,75 |
| Propylenglykol | 6,75 | 6,75 | 6,75 | 6,75 | 6,75 | 6,75 |
| Lorol techn. ¹ | 8,0 | 8,0 | 8,0 | 8,0 | 8,0 | 8,0 |
| Texapon NSO UP ² | 3,5 | 3,5 | 3,5 | 3,5 | 3,5 | 3,5 |
| Dehydol LS2 Deo N ³ | 4,9 | 4,9 | 4,9 | 4,9 | 9,9 | 9,9 |
| Eumulgin CS 15 | 5,0 | 5,0 | - | - | - | - |
| Eumulgin CS 50 | - | - | 5,0 | 5,0 | - | - |
| Arginin | 1,0 | 1,0 | 1,0 | 1,0 | 1,0 | 1,0 |
| Gluadin W40 | 3,0 | 3,0 | 3,0 | 3,0 | 3,0 | 3,0 |
| Ethanolamin | 5,0 | 5,0 | 5,0 | 5,0 | 5,0 | 5,0 |
| (NH₄)₂SO₄ | 1,35 | 2,0 | 1,35 | 2,0 | 1,25 | 1,35 |
| Na₂SO₃ | 0,2 | 0,2 | 0,2 | 0,2 | 0,2 | 0,2 |
| Etidronsäure | 0,24 | 0,24 | 0,24 | 0,24 | 0,24 | 0,24 |
| Ascorbinsäure | 0,4 | 0,4 | 0,4 | 0,4 | 0,4 | 0,4 |
| Wasser | ad 100 | ad 100 | ad 100 | ad 100 | ad 100 | ad 100 |

Die Vergleichsrezeptur V1 enthält eine nicht erfindungsgemäße Menge des Elektrolyts und keinen erfindungsgemäßen Fettalkohol mit einem Ethoxylierungsgrad von 15 bis 100 Mol Ethylenoxid. Es wird nach der Lagerung keine Phasentrennung beobachtet.

Die Vergleichsrezeptur V2 enthält die erfindungsgemäße Menge an Elektrolyt, jedoch keinen erfindungsgemäßen Fettalkohol mit einem Ethoxylierungsgrad von 15 bis 100 Mol Ethylenoxid. Nach der Lagerung wird eine Trübung des Gels und eine Phasentrennung in zwei übereinanderliegenden Phasen beobachtet.

Die erfindungsgemäßen Rezepturen E1 bis E4 enthalten eine erfindungsgemäße Menge des Elektrolyts und einen erfindungsgemäßen Fettalkohol mit einem Ethoxylierungsgrad von 15 bis 100 Mol Ethylenoxid. Alle Rezepturen sind lagerstabil. Sie bleiben nach der Lagerung klar und trennen sich nicht in mehrere Phasen auf.

## Patentansprüche

1. Gelförmige Zusammensetzung, enthaltend
- 6,0 bis 15 Gew.-% eines gesättigten oder ungesättigten, linearen oder verzweigten Alkohols mit 8 bis 36 C-Atomen,
- 0,1 bis 15 Gew.-% einer flüssigen Fettsäuren mit 16 bis 22 C-Atomen in Form einer wasserlöslichen Seife,
- 0,5 bis 10 Gew.-% eines Anlagerungsproduktes von 1 bis 5 Mol Ethylenoxid an einen linearen Fettalkohol mit 8 bis 22 C-Atomen,
- 0,5 bis 10 Gew.-% eines Anlagerungsproduktes von 15 bis 100 Mol Ethylenoxid an einem linearen Fettalkohol mit 8 bis 22 C-Atomen und
- 0 bis 20 Gew.-% eines niedermolekularen wasserlöslichen Alkohols
- nicht weniger als 12 mmol eines Elektrolyts, bezogen auf 100 g der gelförmigen Zusammensetzung,
**dadurch gekennzeichnet, dass** sie eine Viskosität von höchstens 100 mPa s, gemessen bei 20 °C mit einem Rotationsviskosimeter Brookfield, Typ RTV, Spindel #4, bei 4 rpm, besitzt.

2. Gelförmige Zusammensetzung nach Anspruch 1, **dadurch gekennzeichnet, dass** sie als gesättigten oder ungesättigten, linearen oder verzweigten Alkohol mit 8 bis 36 C-Atomen technische Fettalkohole mit 12 bis 18 Kohlenstoffatomen wie beispielsweise Kokos-, Palm-, Palmkern- oder Talgfettalkohol enthält.

3. Gelförmige Zusammensetzung nach Anspruch 1 oder 2, **dadurch gekennzeichnet, dass** sie ein Polyol enthält.

4. Gelförmige Zusammensetzung nach einem der Ansprüche 1 bis 3, **dadurch gekennzeichnet, dass** sie zusätzlich ein wasserlösliches synthetisches Tensid, ausgewählt aus der Gruppe der kationischen, amphoteren, zwitterionischen Tenside und der Fettalkylglycoside, enthält.

5. Gelförmige Zusammensetzung nach einem der Ansprüche 1 bis 4, **dadurch gekennzeichnet, dass** sie zusätzlich Dicetylstearylether, Dicetylstearyloxiethylether, Dicetylstearyldioxiethylether und/oder N,N-Bis(2-Cetylstearyloxiethyl)-aminoethanol enthält.

6. Gelförmige Zusammensetzung nach einem der Ansprüche 1 bis 5, **dadurch gekennzeichnet, dass** sie zusätzlich mindestens ein Oxidationsfarbstoffvorprodukt vom Entwicklertyp und/oder Kupplertyp enthält.

7. Gelförmige Zusammensetzung nach einem der Ansprüche 1 bis 6, **dadurch gekennzeichnet, dass** sie als Elektrolyt mindestens ein Oxidationsfarbstoffvorprodukt vom Entwicklertyp und/oder Kupplertyp enthält, wobei dieses in Form des physiologisch verträglichen Salzes vorliegt, enthält.

8. Gelförmige Zusammensetzung nach einem der Ansprüche 1 bis 7, **dadurch gekennzeichnet, dass** sie zusätzlich ein Protein bzw. Proteinhydrolysat, ausgewählt aus der Gruppe, die gebildet wird aus Erbsen, Soja und Mandelproteinhydrolysate, Akazienprotein sowie Collagen und Keratinhydrolysat, enthält.

9. Gelförmige Zusammensetzung nach einem der Ansprüche 1 bis 8, **dadurch gekennzeichnet, dass** sie zusätzlich ein kationisches Polymer enthält.

10. Gelförmige Zusammensetzung nach einem der Ansprüche 1 bis 9, **dadurch gekennzeichnet, dass** sie zusätzlich mindestens einen direktziehenden Farbstoff enthält.

11. Haarfärbemittel, welche unmittelbar vor dem Aufbringen auf das Haar aus einem fließfähigen Trägermedium, enthaltend mindestens ein Oxidationsfarbstoffvorprodukt vom Entwicklertyp und/oder Kupplertyp, und einer fließfähigen, wässrigen Oxidationsmittelzubereitung miteinander im Gewichtsverhältnis 1:2 bis 2:1 zu einem gelförmigen Färbeansatz vermischt werden, **dadurch gekennzeichnet, dass** das fließfähige Trägermedium eine gelförmige Zusammensetzung nach einem der Ansprüche 1 bis 10 ist.

12. Haarfärbemittel nach Anspruch 11, **dadurch gekennzeichnet, dass** die Oxidationsmittelzubereitung
- 3 bis 9 Gew.-% Wasserstoffperoxid,
- 0,1 bis 5 Gew.-% eines wasserlöslichen synthetischen Tensids und
- 1 bis 5 Gew.-% eines dispergierten Acrylsäure und/oder Methacrylsäure-polymerisats oder -Copolymerisats
enthält.

13. Verfahren zur Herstellung eines Haarfärbemittel, bei welchem eine Zusammensetzung nach einem der Ansprüche 1 bis 10, enthaltend mindestens ein Oxidationsfarbstoffvorprodukt vom Entwicklertyp und/oder Kupplertyp, und eine Oxidationsmittelzubereitung miteinander im Gewichtsverhältnis 1:2 bis 2:1 zu einem gelförmigen Färbeansatz vermischt werden.

## Claims

1. A composition in gel form, containing
- 6.0 to 15 wt.% of a saturated or unsaturated, linear or branched alcohol with 8 to 36 C atoms,
- 0.1 to 15 wt.% of a liquid fatty acid with 16 to 22 C atoms in the form of a water-soluble soap,
- 0.5 to 10 wt.% of an addition product of 1 to 5 mol of ethylene oxide onto a linear fatty alcohol with 8 to 22 C atoms,
- 0.5 to 10 wt.% of an addition product of 15 to 100 mol of ethylene oxide onto a linear fatty alcohol with 8 to 22 C atoms and
- 0 to 20 wt.% of a low molecular weight water-soluble alcohol
- no less than 12 mmol of an electrolyte, relative to 100 g of the composition in gel form,
**characterised in that** it has a viscosity of at most 100 mPa·s, measured at 20°C with a Brookfield rotational viscometer, model RTV, spindle #4, at 4 rpm.

2. A composition in gel form according to claim 1, **characterised in that** it contains technical fatty alcohols with 12 to 18 carbon atoms, such as for example coconut, palm, palm kernel or tallow fatty alcohol, as the saturated or unsaturated, linear or branched alcohol with 8 to 36 C atoms.

3. A composition in gel form according to claim 1 or claim 2, **characterised in that** it contains a polyol.

4. A composition in gel form according to any one of claims 1 to 3, **characterised in that** it additionally contains a water-soluble synthetic surfactant, selected from the group of cationic, amphoteric, zwitterionic surfactants and of fatty alkyl glycosides.

5. A composition in gel form according to any one of claims 1 to 4, **characterised in that** it additionally contains dicetylstearyl ether, dicetylstearyl oxyethyl ether, dicetylstearyl dioxyethyl ether and/or N,N-bis(2-cetylstearyl oxyethyl)-aminoethanol.

6. A composition in gel form according to any one of claims 1 to 5, **characterised in that** it additionally contains at least one oxidation dye precursor of the developer and/or coupler type.

7. A composition in gel form according to any one of claims 1 to 6, **characterised in that** it contains as the electrolyte at least one oxidation dye precursor of the developer and/or coupler type, said precursor being present in the form of a physiologically acceptable salt.

8. A composition in gel form according to any one of claims 1 to 7, **characterised in that** it additionally contains a protein or protein hydrolysate selected from the group made up of pea, soy and almond protein hydrolysates, acacia protein together with collagen and keratin hydrolysate.

9. A composition in gel form according to any one of claims 1 to 8, **characterised in that** it additionally contains a cationic polymer.

10. A composition in gel form according to any one of claims 1 to 9, **characterised in that** it additionally contains at least one direct dye.

11. Hair dyeing agents which are prepared immediately before application onto the hair by mixing a flowable carrier medium, containing at least one oxidation dye precursor of the developer and/or coupler type, and a flowable, aqueous oxidising agent preparation with one another in a 1:2 to 2:1 weight ratio to yield a dyeing batch in gel form, **characterised in that** the flowable carrier medium is a composition in gel form according to any one of claims 1 to 10.

12. Hair dyeing agents according to claim 11, **characterised in that** the oxidising agent preparation contains
- 3 to 9 wt.% of hydrogen peroxide,
- 0.1 to 5 wt.% of a water-soluble synthetic surfactant and
- 1 to 5 wt.% of a dispersed acrylic acid and/or methacrylic acid polymer or copolymer.

13. A method for producing a hair dyeing agent, in which a composition according to any one of claims 1 to 10, containing at least one oxidation dye precursor of the developer and/or coupler type, and an oxidising agent preparation are mixed with one another in a 1:2 to 2:1 weight ratio to yield a dyeing batch in gel form.

## Revendications

1. Composition en forme de gel, contenant
- 6,0 à 15% en poids d'un alcool, saturé ou insaturé, linéaire ou ramifié, comprenant 8 à 36 atomes de carbone,
- 0,1 à 15% en poids d'un acide gras liquide comprenant 16 à 22 atomes de carbone sous forme d'un savon soluble dans l'eau,
- 0,5 à 10% en poids d'un produit d'addition de 1 à 5 moles d'oxyde d'éthylène sur un alcool gras linéaire comprenant 8 à 22 atomes de carbone,
- 0,5 à 10% en poids d'un produit d'addition de 15 à 100 moles d'oxyde d'éthylène sur un alcool gras linéaire comprenant 8 à 22 atomes de carbone et
- 0 à 20% en poids d'un alcool de bas poids moléculaire soluble dans l'eau
- pas moins de 12 mmoles d'un électrolyte, par rapport à 100 g de la composition en forme de gel,
**caractérisée en ce qu'**elle présente une viscosité d'au plus 100 mPa.s, mesurée à 20°C avec un viscosimètre de rotation Brookfield, type RTV, mobile #4, à 4 t/min.

2. Composition en forme de gel selon la revendication 1, **caractérisée en ce qu'**elle contient, comme alcool saturé ou insaturé, linéaire ou ramifié, comprenant 8 à 36 atomes de carbone des alcools gras de qualité technique comprenant 12 à 18 atomes de carbone tels que par exemple l'alcool gras de coco, de palme, de noyau de palme ou de suif.

3. Composition en forme de gel selon la revendication 1 ou 2, **caractérisée en ce qu'**elle contient un polyol.

4. Composition en forme de gel selon l'une quelconque des revendications 1 à 3, **caractérisée en ce qu'**elle contient en outre un agent tensioactif synthétique, soluble dans l'eau, choisi dans le groupe des agents tensioactifs cationiques, amphotères, zwittérioniques et des alkylglycosides gras.

5. Composition en forme de gel selon l'une quelconque des revendications 1 à 4, **caractérisée en ce qu'**elle contient en outre du dicétylstéaryléther, du dicétylstéaryloxyéthyléther, du dicétylstéaryldioxyéthyléther et/ou du N,N-bis(2-cétylstéaryloxyéthyl)-aminoéthanol.

6. Composition en forme de gel selon l'une quelconque des revendications 1 à 5, **caractérisée en ce qu'**elle contient en outre au moins un précurseur d'un colorant par oxydation du type agent de développement et/ou du type agent de couplage.

7. Composition en forme de gel selon l'une quelconque des revendications 1 à 6, **caractérisée en ce qu'**elle contient comme électrolyte au moins un précurseur d'un colorant par oxydation du type agent de développement et/ou du type agent de couplage, celui-ci se trouvant sous forme du sel physiologiquement acceptable.

8. Composition en forme de gel selon l'une quelconque des revendications 1 à 7, **caractérisée en ce qu'**elle contient en outre une protéine ou, selon le cas, un hydrolysat de protéines, choisi(e) dans le groupe formé par les hydrolysats de protéines de pois, de soja et d'amande, les protéines d'acacia ainsi que le collagène et l'hydrolysat de kératine.

9. Composition en forme de gel selon l'une quelconque des revendications 1 à 8, **caractérisée en ce qu'**elle contient en outre un polymère cationique.

10. Composition en forme de gel selon l'une quelconque des revendications 1 à 9, **caractérisée en ce qu'**elle contient en outre au moins un colorant direct.

11. Agents de teinture pour cheveux, constitués par un agent support pouvant s'écouler, contenant au moins un précurseur d'un colorant d'oxydation du type agent de développement et/ou du type agent de couplage, et une préparation pouvant s'écouler, aqueuse d'un oxydant, qui sont mélangés immédiatement avant l'application sur les cheveux l'un avec l'autre dans un rapport pondéral de 1:2 à 2:1 en une charge colorante en forme de gel, **caractérisés en ce que** l'agent support pouvant s'écouler est une composition en forme de gel selon l'une quelconque des revendications 1 à 10.

12. Agents de teinture de cheveux selon la revendication 11, **caractérisés en ce que** la préparation d'un oxydant contient
- 3 à 9% en poids de peroxyde d'hydrogène,
- 0,1-5% en poids d'un agent tensioactif synthétique, soluble dans l'eau et
- 1 à 5% en poids d'un polymère ou d'un copolymère dispersé d'acide acrylique et/ou méthacrylique.

13. Procédé pour la préparation d'un agent de teinture pour cheveux, dans lequel une composition selon l'une quelconque des revendications 1 à 10, contenant au moins un précurseur d'un colorant par oxydation du type agent de développement et/ou du type agent de couplage et une préparation d'un oxydant sont mélangées l'une avec l'autre dans un rapport pondéral de 1:2 à 2:1 en une charge colorante en forme de gel.
